(19) Europäisches Patentamt
European Patent Office
Office européen des brevets

(11) Publication number: **0 049 728**
**B1**

(12) **EUROPEAN PATENT SPECIFICATION**

(45) Date of publication of patent specification: **12.11.86**

(51) Int. Cl.$^4$: **A 23 K 1/16,** C 07 C 91/40

(21) Application number: **81100368.0**

(22) Date of filing: **19.01.81**

(54) **Method for enhancing the growth rate of meat-producing animals and for improving the lean meat to fat ratio thereof.**

(30) Priority: **25.08.80 US 181254**
**25.08.80 US 181255**

(43) Date of publication of application:
**21.04.82 Bulletin 82/16**

(45) Publication of the grant of the patent:
**12.11.86 Bulletin 86/46**

(84) Designated Contracting States:
**BE CH DE FR GB IT LI NL SE**

(56) References cited:
**EP-A-0 008 715**
**EP-A-0 026 298**
**DE-A-2 804 625**
**GB-A-1 141 606**
**GB-A-1 218 135**
**US-A-3 536 712**

(73) Proprietor: **AMERICAN CYANAMID COMPANY**
**1937 West Main Street P.O. Box 60**
**Stamford Connecticut 06904 (US)**

(72) Inventor: **Baker, Pamela Koenig**
**62 Hart Avenue**
**Hopewell New Jersey (US)**
Inventor: **Kiernan, Jane Ann**
**620 Madison Avenue**
**Dunellen New Jersey (US)**

(74) Representative: **Wächtershäuser, Günter, Dr.**
**Tal 29**
**D-8000 München 2 (DE)**

**The file contains technical information submitted after the application was filed and not included in this specification**

**EP 0 049 728 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Substitution products of 1-(amino-dihalophenyl)-2-aminoethanes, and the acid addition salts thereof, are disclosed in United States Patent 3,536,712. Specifically, methods for the synthesis of said compounds are disclosed as useful for enhancing the blood circulation, and as bronchodilators, analgesics, sedatives, antipyretics, antiphlogistics and antitussives in warm-blooded animals. However, only the analgesic utility is examplified. The preparation of other related 1-(amino-dihalophenyl)-2-aminoethanols and their derivatives are disclosed in JP—A 77 83,619 (Chemical Abstracts, *87,* 201061r), German Offenlegungsschrift 2,804,625 (1979), German Offenlegungsschrift 2,157,040 (1973), German Offenlegungsschrift 2,261,914 (1974), European Patent Application 8,715 (1980), Netherlands Patent Application 7,303,612 (1973) and German Offenlegungsschrift 28 04 625. These applications disclose uses selected from analgesics, broncholytic, antiinflammatory, uterine spasmolytic, β-blocking activities, antispactic activity on cross-striped muscle structure, for tocology, reducing blood pressure by peripheral vasodilation and mobilizing body fat, and for treating allergies. There is no indication or suggestion in any of these disclosures that said compounds are effective as growth-promoting agents for meat-producing animals, such as poultry, cattle, sheep or the like; nor is there any suggestion that said compounds improve the efficiency of feed utilization by said meat-producing animals or the ratio of lean meat to fat.

US—A—3818101 describes a method for increasing the feed intake of meat producing animals beyond satiation by administering hydroxphenethanolamines with one or two hydroxyl groups attached to the phenyl group. These compounds lead to an accelerated growth rate. These compounds do not reduce the ratio of fat to lean meat or the even increase said ratio.

Therefore, it is the problem of the present invention to provide a method for increasing the ratio of lean meat to fat of meat producing animals.

In accordance with the process of the invention, it has been found that the growth rate of meat-producing animals such as chickens, turkeys, rabbits, sheep, swine, goats and cattle, including calves, can be increased, the efficiency of feed utilization thereby measurably improved, and the lean meat to fat ratio improved by the oral or parenteral administration to said animals of an effective amount of a compound of the following formula at selected levels of administration

(I) 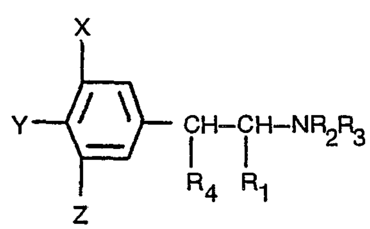

wherein

X is hydrogen or halogen;

Y is hydrogen, $NR_8R_9$ or $NHCOR_5$;

Z is halogen, CN, $CF_3$, $COOR_1$, $CONH_2$, methyl, methoxy or $NO_2$;

$R_1$ is hydrogen, $C_{1-4}$alkyl;

$R_2$ is hydrogen, $C_{1-6}$alkyl or $C_3$—$C_4$-alkenyl;

$R_3$ is hydrogen $C_1$—$C_6$alkyl, $C_3$—$C_6$cycloalkyl, $C_3$—$C_4$-alkenyl, phenyl, 2-hydroxyethyl, α,α-dimethylphenethyl or benzyl;

$R_4$ is OH, $OR_6$ or $SR_{11}$;

$R_5$ is hydrogen, $C_1$—$C_4$-alkyl, $C_1$—$C_4$-alkoxy or

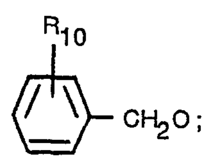;

$R_6$ is $C_1$—$C_6$alkyl, $C_2$—$C_5$ alkanoyl,

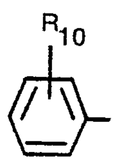 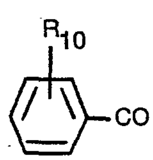 or 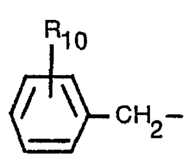 or $C_3$—$C_4$-alkenyl;

$R_8$ is hydrogen, $C_1$—$C_4$-alkyl or $C_3$—$C_4$-alkenyl;
$R_9$ is hydrogen, $C_1$—$C_6$ alkyl, $C_4$—$C_6$ cycloalkyl or $C_3$—$C_4$ alkenyl;
$R_{10}$ is chloro, dichloro, methyl, dimethyl, methoxy, dimethoxy or nitro;
$R_{11}$ is $C_1$—$C_6$-alkyl, phenyl, or benzyl;

with the privosos that

when $R_3$ is phenyl, 2-hydroxyethyl, α,α-dimethylphenethyl, $C_3$—$C_6$ cycloalkyl or benzyl $R_2$ is hydrogen; and

when $R_3$ is 2-hydroxyethyl, $R_4$ is hydroxyl; and

when $R_6$ is alkanoyl or

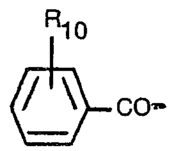

$R_2$ and $R_3$ are substituents other than hydrogen, except when $R_3$ is an alkyl or a substituted alkyl group which contains a tertiary carbon attached to the nitrogen; and

when Y is hydrogen, X and Z are halogen, and $R_2$ is hydrogen and $R_3$ is isopropyl, 2-butyl or *t*-butyl; and

when $R_8$ is $C_1$—$C_4$ alkyl or $C_3$—$C_4$ alkenyl, $R_9$ is hydrogen, $C_1$—$C_4$ alkyl or $C_3$—$C_4$ alkenyl; and that at least one of X and Y represents a substituent other than hydrogen; and

when Z is a group other than halogen, Y is $NR_8R_9$ or $NHCOR_5$; and

further provided that when Y is hydrogen, $NH_2$ or $NHCOR_5$, and Z is halogen then $R_4$ cannot be OH or $OR_6$, wherein $R_6$ is $C_1$—$C_6$ alkyl;

racemic mixtures of the above identified compounds and the optically active isomers, and non-toxic, pharmacologically acceptable acid addition salts thereof.

Applicants prior European patent application 80 10 46 19.4 describes similar compounds for use in a method for improving the ratio of lean meat to fat. These compounds and the compounds used in the present invention do not only enhance the growth rate but also increase the ratio of lean meat to fat and improve the feed utilization efficiency of meat producing animals.

A preferred group of compounds for use in the method of this invention have the above formula I structure wherein X is hydrogen, chlorine, or bromine; Y is hydrogen or $NR_8R_9$; Z is chlorine, bromine, CN, $CF_3$; $R_1$ is hydrogen or methyl; $R_4$ is OH, $OR_6$, $SR_{11}$; $R_6$ is $C_1$—$C_4$ alkyl, or $C_2$—$C_5$ alkanoyl, or a non-toxic, pharmacologically acceptable acid addition salt thereof.

The most preferred compounds for use in this invention are: N - *tert* - butyl - 3,5 - dichloro - β-methoxy - 4 - methylaminophenethylamine; α - [(*tert* - butylamino)methyl] - 3,5 - dichloro - 4 - isopropylaminobenzyl alcohol; 5 - (2 - (*tert* - butylamino) - 1 - hydroxyethyl] - 3 - chloroanthranilonitrile; 5 - [2 - (*tert* - butylamino) - 1 - hydroxyethyl]anthranilonitrile; methyl - 5 - [2 - (*tert* - butylamino) - 1 - hydroxyethyl) - 3 - chloroanthranilate; 4 - amino - N - *tert* - butyl - 3,5 - dichloro - β - (methylthio)phenethylamine; α - [(*tert* - butylamino) - methyl] - 3,5 - dichloro-4-methylaminobenzyl alcohol; α - [(*tert* - butylamino)methyl - 3,5 - dichloro - 4 - dimethylaminobenzyl alcohol; methyl - 4 - [2 - (*tert* - butylamino) - 1 - hydroxyethyl] - 2,6 - dichlorocarbanilate; and the non-toxic, pharmaceutically acceptable acid addition salts thereof.

It is found, that formula (I) compounds below (wherein Y is hydrogen) can be prepared by the condensation of an appropriately substituted styrene oxide with the appropriately substituted amine in the presence of an inert solvent, such as a lower alcohol at or near the boiling point of same, as shown below:

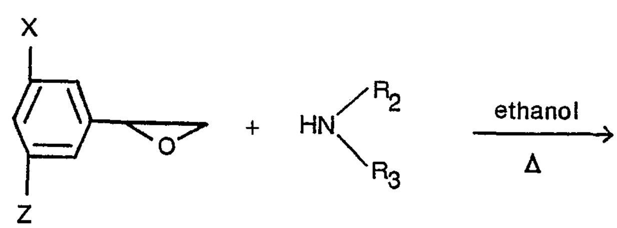


(I)

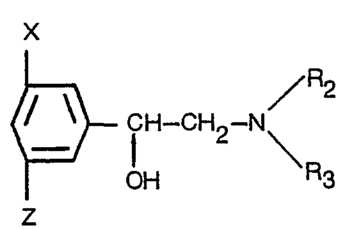

wherein X and Z are halogen, $R_2$ and $R_3$ are as hereinabove defined and Y is hydrogen. Thus, 3,5-dichlorostyrene oxide can be reacted with an equimolar or molar excess of *t*-butylamine in ethanol at reflux from about one to about eight hours, or until the reaction is essentially complete and the desired α-[(*t*-butylamino)methyl]-3,5-dichlorobenzyl alcohol is obtained as illustrated below:

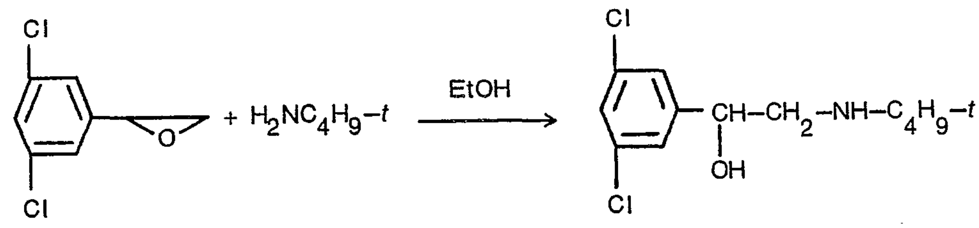


The thus obtained product can be purified by known procedures, such as chromatography or recrystallization of salts thereof.

The above styrene oxide is made by reducing the corresponding phenacyl bromide with $NaBH_4$ at or below 5°C in the presence of an anhydrous lower alcohol, such as ethanol. The phenacyl bromide intermediate is prepared by brominating the appropriately substituted acetophenone with $CuBr_3$ in the presence of chloroform and ethyl acetate. The above sequence may be graphically illustrated as follows:

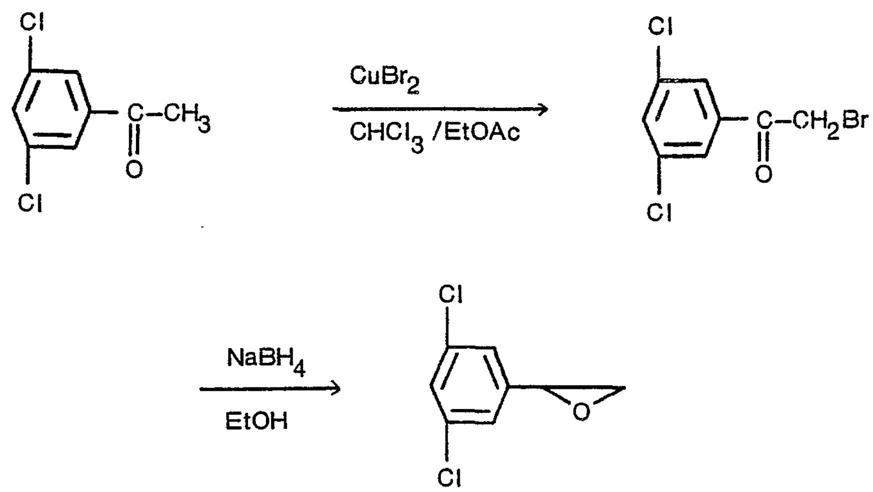


Alternatively, a formula (I) compound wherein Y is hydrogen may be prepared from the corresponding formula (I) compound wherein Y is amino, *via* a deamination reaction, by dissolving the amine in 50—52% aqueous hypophosphorous acid ($H_3PO_2$). The solution is chilled below 10°C, and an equimolar or excess amount of sodium nitrite is added to an aqueous solution with stirring over a period of time. On completion of the addition, the reaction mixture is warmed to room temperature and stirred for an additional period of time. The product is then recovered from the reaction mixture by standard laboratory procedures and purified if so desired.

The preparation of 4-substituted aminoacetophenones required for the preparation of 4-substituted phenylethane derivatives which are now found to be useful for raising meat-producing animals, is exemplified as follows:

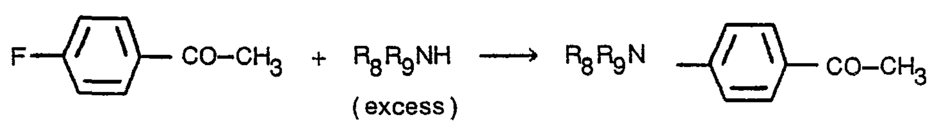


The fluorine displacement is carried out with excess amine in the presence or absence of a solvent; and, if a solvent is required, water appears to be the most useful. With volatile amines, the reaction is conducted in a sealed vessel and generally temperatures of 50—100°C are sufficient to complete the reaction.

Chlorination and bromination of these aminoacetophenones may be conducted with N-chlorosuccinimide and Npbromosuccinimide in toluene, chlorobenzene or dichlorobenzene at 90—100°C. Iodination may be conducted with NaI/N,N-dichlorobenzenesulfonamide or iodine monochloride in acetic acid.

By reacting these acetophenones with bromine in chloroform or methylene chloride, the corresponding phenacyl bromides are prepared. These phenacyl bromides are then reacted with $R_2R_3N$ amines and the aminoketones are reduced with $NaBH_4$ or $NaCNBH_3$ by conventional techniques described in reference cited hereinbefore. Naturally, compounds which contain groups reactive to halogen, such as when $R_8$ is alkenyl, require other approaches that are discussed below.

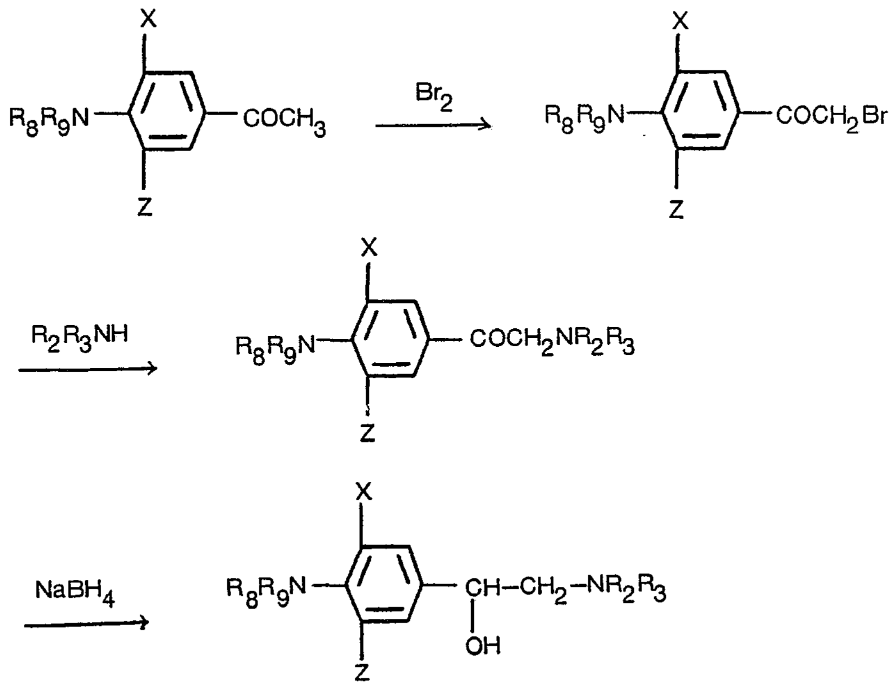


wherein X and Z are hydrogen, chlorine, or bromine and $R_2$ and $R_3$ are hydrogen, $C_1$—$C_4$ alkyl, or $C_2$—$C_3$ alkenyl groups.

The compounds of formula I, wherein $R_8$ and $R_9$ are groups other than both being hydrogen are also prepared by the following general scheme:

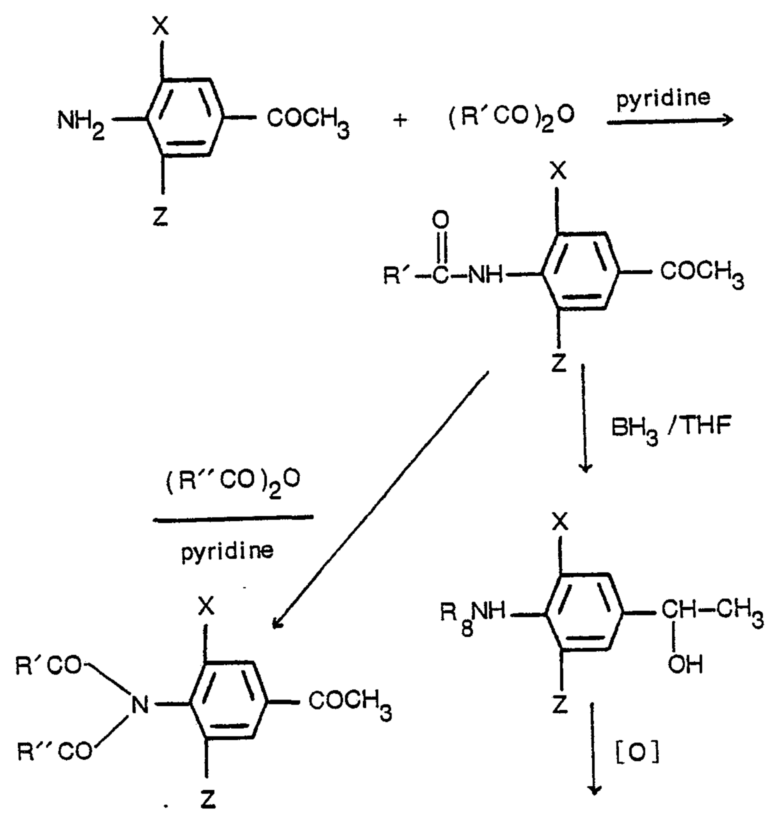

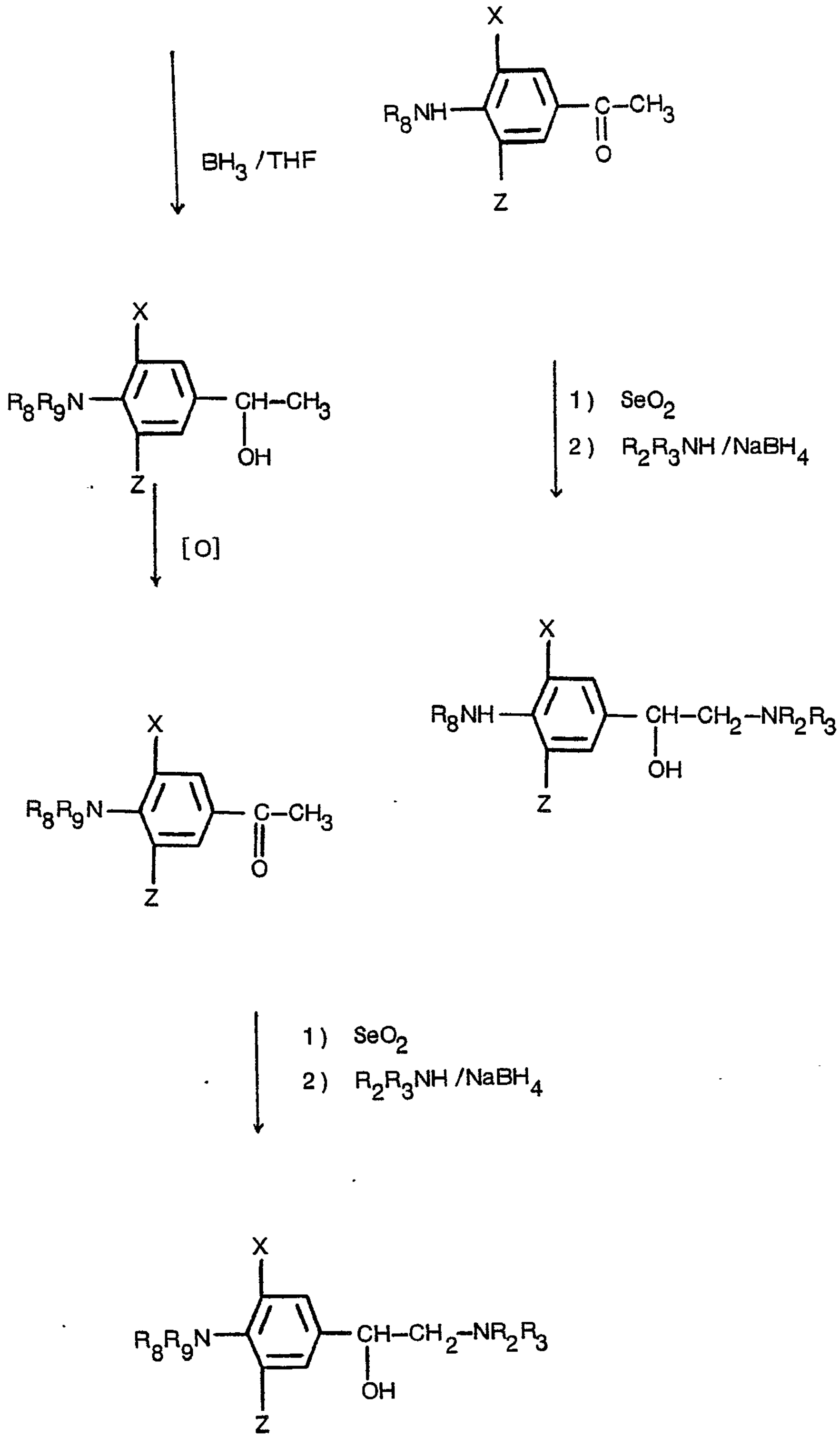


The methods utilized in the above scheme are either reported in references cited thereinbefore or by conventional methods. Oxidation of the alcohol may be conducted with chromic acid (Jones Reagent), $MnO_2$, pyridinium chlorochromate, or other oxidizing agents. Where X or Z are the $BH_3$-reducible groups, CN, COOR, or $CONH_2$, the appropriate acetophenones are prepared by displacement of X or Z represented by bromine with CuCN/DMF at 100—160°C by the conventional method, after reduction of the acylated aminoacetophenones in the first step followed by re-oxidation in the second step of the above procedure. The cyano substituted-amino-acetophenones are then converted to their corresponding ethanolamines, which are then converted to the desired esters, acids, and amides by conventional methods, such as $R_1OH$/acid→esters, hydrolyses→acids and partial hydrolyses→amides.

Furthermore, compounds of the following structure are prepared by allowing the corresponding ethanolamines to react with an equivalent or slight excess of the acid anhydrides with or without organic bases such as tertiary amines or pyridine.

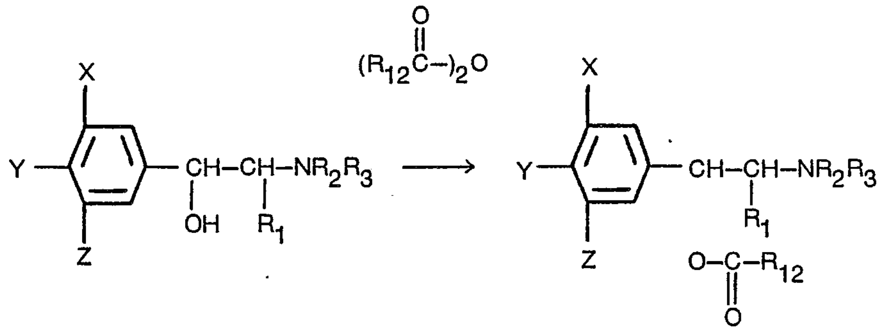

wherein $R_{12}$ is $C_1$—$C_4$-alkyl or

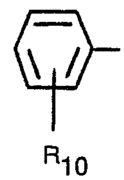

The reactions are conducted in inert solvents such as chlorinated hydrocarbons, or aromatic solvents at 0—25°C. Reaction of the anhydride at the hydroxyl group proceeds well provided $R_2$ and $R_3$ are groups other than hydrogen and when $R_2$ is hydrogen, $R_3$ is a substituent containing a tertiary carbon attached to nitrogen.

Additionally, Formula I compounds, wherein $R_8$ and $R_9$ are selected from hydrogen and $C_3$—$C_4$ alkenyl, are prepared by alkenylation of 4-amino-3,5-disubstituted phenacyl bromides in dimethylformamide (DMF) in the presence of an acid acceptor, such as triethylamine or sodium carbonate, at 70—100°C to afford mono and dialkenylated products which are separated and converted to I by conventional methods. The following scheme illustrates above-described general method:

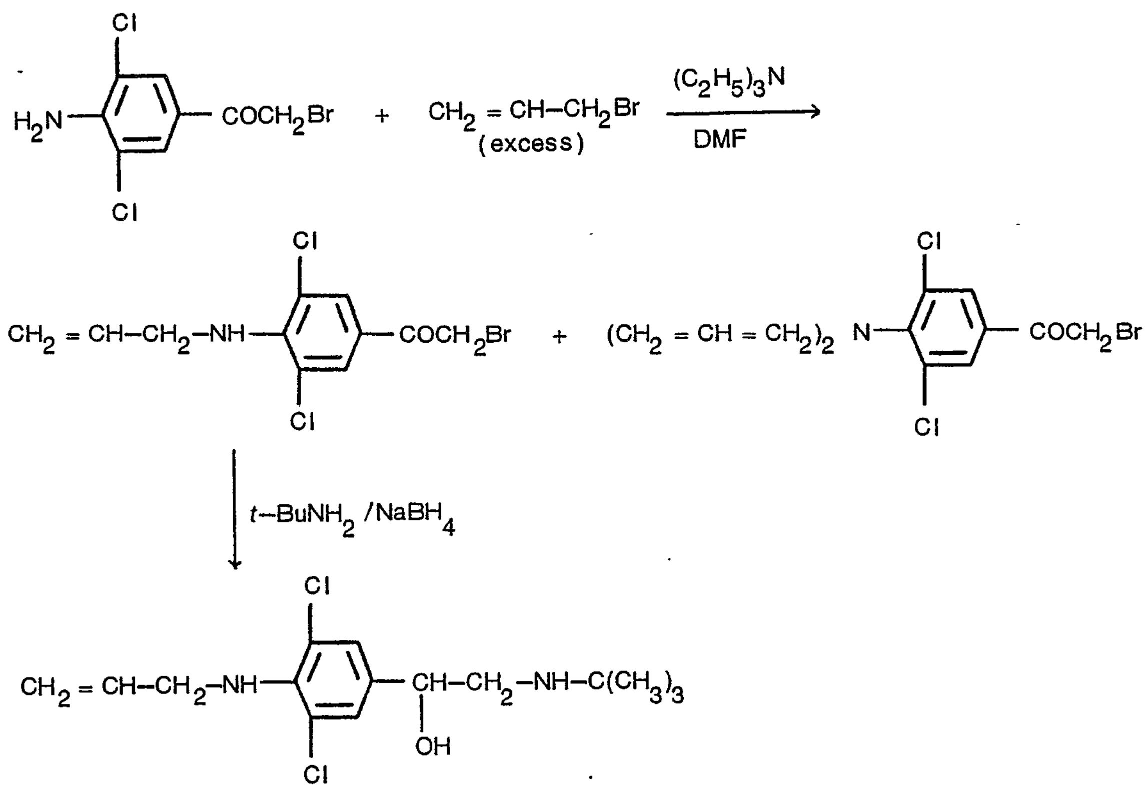

Formula (I) compounds wherein $R_4$ is $OR_6$ and $SR_{11}$, wherein $R_6$ and $R_{11}$ are as hereinabove defined, may be prepared by converting the alcohol ($R_4$—OH) with thionyl chloride under an inert blanket of gas such as nitrogen at a temperature range of from about 0 to 10°C and preferably at 0 to 5°C for a reaction period sufficient to essentially complete the reaction. The thus obtained halo compound is isolated by conventional methods and is then reacted with the appropriate alcohol or mercaptan, under an inert blanket of gas, such as nitrogen at a temperature range of from about 0 to 50°C. The formula (I) product thus obtained is then isolated by standard laboratory methods and purified, if so desired. The above reaction sequence may be graphically illustrated as follows:

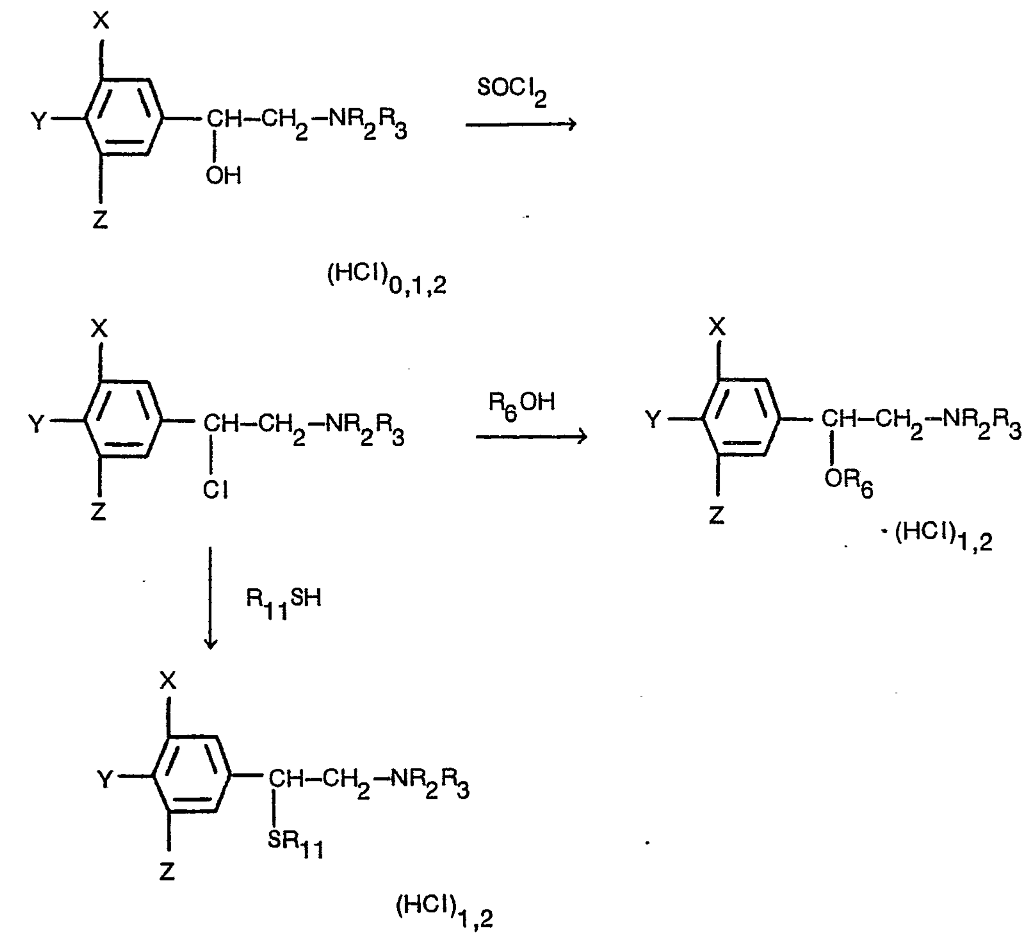


wherein X, Y, Z, $R_2$, $R_3$, $R_6$ and $R_{11}$ are as hereinabove defined.

These displacement reactions may also be performed by using an excess of alkoxide $R_6O^-$) or mercaptide ($R_{11}S^-$) in an inert solvent such as tetrahydrofuran to afford the above esters and thioesters in a similar manner.

Alternatively, a formula (I) compound wherein $R_4$ is $OR_6$ may be prepared by dissolving the corresponding formula (I) compound wherein $R_4$ is OH in the corresponding $R_6OH$ alcohol and saturating the thus obtained solution with dry HCl gas. The reaction mixture is then stirred at room temperature for a period of time sufficient to essentially complete the reaction and the product is then isolated by standard laboratory procedures and purified, if so desired. This reaction sequence may be illustrated as follows:

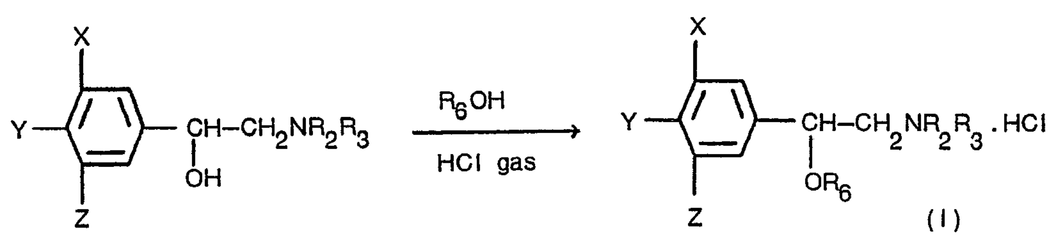


(I)

wherein X, Y, Z, $R_2$, $R_3$ and $R_6$ are as hereinabove defined.

In the present specification and claims, the term α,α-dimethylphenethyl means a structure having the following configuration:

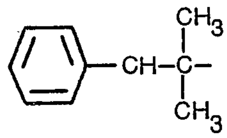

When orally administered in the feed, generally about 0.01 to 300 grams per ton of feed of the above-identified phenylethane derivative or acid addition salt thereof, is effective for enhancing the growth rate and improving the efficiency of feed utilization by the above-mentioned meat-producing animals.

Since the effective and preferred dietary levels of the active ingredient vary somewhat from species to species in the above-mentioned animals, said levels for each animal species are listed in Table I below on a gram per ton feed basis:

TABLE I

| Compound | Effective Feed Level g/1.100 kg | Preferred Level g/1.100 kg | Animal Animal |
|---|---|---|---|
| Formula (I) | 0.1—200 | 1—100 | Sheep, Goats |
| | 0.01—50 | 0.1—10 | Chickens, Rabbits |
| | 0.01—50 | 0.1—10 | Turkeys |
| | 0.1—300 | 1—100 | Cattle & Swine |

Animal feed compositions which will provide the desired group promotion and feed efficiency in the above-mentioned animals can be prepared by admixing the phenylethane derivative or acid addition salt thereof, or an animal feed supplement containing said compound, with a sufficient quantity of an appropriate animal feed to provide the desired level of active compound in said feed.

Animal feed supplements can be prepared by admixing about 10% to 75% by weight of the phenylethane derivative of acid addition salt thereof, with about 90% to 25% by weight of a suitable carrier or diluent. Carriers suitable for use to make up the feed supplement compositions include the following: alfalfa meal, soybean meal, cottonseed oil meal, linseed oil meal sodium chloride, cornmeal, can molasses, urea, bone meal, corncob meal and the like. The carrier promotes a uniform distribution of the active ingredient in the finished feed into which the supplement is blended. It thus performs an important function by ensuring proper distribution of the active ingredient throughout the feed.

If the supplement is used as a top dressing for feed, it likewise helps to ensure uniformity of distribution of the active material across the top of the dressed feed.

For parenteral administration, the phenylethane derivative may be prepared in the form of a paste or pellet and administered as an implant, usually under the skin of the head or ear of the animal in which enhanced growth rate and/or improved efficiency of feed utilization is sought.

In practice, parenteral administration generally involves injection of a sufficient amount of the above-said phenylethane derivative to provide the animal with from 0.001 to 50 mg/kg of body weight of the active ingredient. The preferred dosage level for cattle is the range of from 0.001 to 25 mg/kg of body weight of the active phenylethane derivative. The preferred dose level of said phenylethane derivative for poultry is about 0.001 to 35 mg/kg of animal body weight and the preferred dose level of said phenylethane derivative for sheep and goats is 0.001 to 40 mg/kg of animal body weight. The preferred dose level for rabbits is 0.001 to 35 mg/kg of animal body weight.

Paste formulations can be prepared by dispersing the active phenylethane derivative in a pharmaceutically acceptable oil such as peanut oil, sesame oil, corn oil or the like.

Pellets containing an effective level of the phenylethane derivative can be prepared by admixing the above-said active ingredient with a diluent such as carbowax, biodegradable polymers, carnauba wax, or the like. A lubricant, such as magnesium stearate or calcium stearate may be added to improved the pelleting process if desired.

It is, of course, recognized that more than one pellet may be administered to an animal to achieve the desired dose level which will provide the increased growth rate and/or improve efficiency of feed utilization by said animal. Moreover, it has been found that additional implants may also be introduced periodically during the treatment period in order to maintain the proper drug release rate in the animal's body.

In addition to enhanced growth promotion and improved efficiency of feed utilization by meat-producing animals, the compounds of the present invention have the added advantage that, at selected levels of administration, they increase the deposition of lean meat (i.e., muscle or protein) in said animals and improve the carcass quality thereof by increasing the ratio of lean meat to fat in the animals receiving them. This biological response has substantial advantage to poultrymen, cattlemen, and swine, sheep and goat producers since administration of said compounds at selected levels yields leaner animals which command premium prices from the meat industry.

These and other advantages of the present invention will become apparent from the examples set forth below.

Example 1

Evaluation of Test Compounds as Animal Growth Promoters

CFI female micve from Carworth Farms are received when they are six weeks old. They are housed ten to a cage in air-conditioned rooms 22,, 2—24.4°C (72°F—76°F) with automatically controlled lights, 14 hours on and 10 hours off. The basal diet used in these studies is Purina Laboratory Chow (see description below), which is supplied ad libitum. Water is allowed *ad libitum*.

Thirteen days after arrival, the mice are weighed in groups of ten and assigned at random to the different treatments. The concentration of the different compounds in the diet is indicated in the following tables. Twelve days later the mice are weighed again, and the experiment terminated. Test data are provided in Table II below wherein data are reported as percentage gain over controls. Different control animals are used for each test. The following is a description of the diet to which the growth-promoting compounds were added.

DIET

Guaranteed Analysis

| | |
|---|---|
| Crude protein not less than | 23.0% |
| Crude fat not less than | 4.5% |
| Crude fiber not more than | 6.0% |
| Ash not more than | 9.0% |

Ingredients

Meat and bone meal, dried skimmed milk, wheat germ meal, fish meal, animal liver meal, dried beet pulp, ground extruded corn, ground oat groats, soybean meal, dehydrated alfalfa meal, can molasses, animal fat preserved with BHA, vitamin $B_{12}$ supplement, calcium pantothenate, choline chloride, folic acid, riboflavin supplement, brewer's dried yeast, thiamin, niacin, vitamin A supplement, D-activated plant sterol, vitamin E supplement, calcium carbonate, dicalcium phosphate, iodized salt, ferric ammonium citrate, iron oxide, manganous oxide, cobalt carbonate, copper oxide, zinc oxide.

TABLE II

Evaluation of Test Compounds as Animal Growth Promoters

| Compound | Dosage (ppm) | Gain (grams) | % Gain Over Controls |
|---|---|---|---|
| α-[(*Tert*-butylamino)methyl]-3,5-dichloro-4-dimethylaminobenzyl alcohol | 200<br>50 | 16.0<br>21.9 | 0<br>+36.9 |
| α-[(*Tert*-butylamino)methyl]-3,5-dichloro-4-methylaminobenzyl alcohol | 200<br>50 | 19.4<br>24.4 | +21.3<br>+52.5 |
| Methyl-4-[2-(*Tert*-butylamino)-1-hydroxyethyl]-2,6-dichlorocarbanilate | 50 | 27.9 | +40.8 |
| 5-[2-(*Tert*-butylamino)-1-hydroxyethyl]-3-chloroanthranilonitrile | 200<br>50 | 27.3<br>26.2 | +90.9<br>+83.2 |
| α-[(*Tert*-butylamino)methyl]-3,5-dichloro-4-isopropylaminobenzyl alcohol | 200<br>50<br>12<br>3 | 24.6<br>24.3<br>28.0<br>27.3 | +72.0<br>+69.9<br>+95.8<br>+90.8 |
| 5-[2-(*Tert*-butylamino)-1-hydroxyethyl]-anthranilonitrile | 200<br>50 | 29.6<br>29.9 | +79.4<br>+81.2 |
| Methyl-5-[2-*Tert*-butylamino)-1-hydroxyethyl]-3-chloroanthranilate hydrochloride | 200<br>50 | 24.4<br>20.1 | +47.9<br>+21.8 |
| 4-Amino-N-*Tert*-butyl-3,5-dichloro-β-(methylthio)-phenethylamine hydrochloride | 200<br>50 | 25.4<br>25.3 | +55.8<br>+55.2 |

TABLE II (Continued)

Evaluation of Test Compounds as Animal Growth Promoters

| Compound | Dosage (ppm) | Gain (grams) | % Gain Over Controls |
|---|---|---|---|
| 3-Bromo-5-[2-(*tert*-butylamino)-1-hydroxyethyl]-anthranilonitrile | 200<br>50 | 16.1<br>24.2 | +50.5<br>+126.2 |
| 4-Amino-α-[(*tert*-butylamino)methyl]-3-methyl-benzyl alcohol | 100 | 20.8 | +94.5 |
| 4-(Butylamino)-α-[(*tert*-butylamino)methyl]-3,5-dichlorobenzyl alcohol | 200<br>50 | 19.3<br>19.4 | +80.4<br>+81.3 |
| 2-Amino-3-bromo-5-[2-(*tert*-butylamino)-1-hydroxyethyl]benzamide | 200<br>50 | 17.4<br>19.8 | +62.6<br>+85.0 |
| 4-Amino-α-[(*tert*-butylamino)methyl]-3,5-dichlorobenzyl alcohol acetate (ester) | 200<br>50 | 14.6<br>19.1 | +36.4<br>+78.5 |
| 3-Bromo-5-[2-*tert*-butylamino)-1-hydroxyethyl]-anthranilic acid | 200<br>50 | 18.2<br>13.6 | +70.1<br>+27.1 |
| N-*tert*-butyl-3,5-dichloro-8-methoxy-4-methyl-aminophenethylamine hydrochloride | 200<br>50 | 18.0<br>23.1 | +68.2<br>+115.9 |
| α-[(*tert*-butylamino)methyl]-3,5-dichloro-4-(hexylamino)benzyl alcohol | 200<br>50 | 19.6<br>20.7 | +83.2<br>+93.5 |
| 4-Amino-α-[(*tert*-butylamino)methyl]-3,5-dichloro-benzyl alcohol acetate, acetic acid salt | 200<br>50 | 14.4<br>18.7 | +34.6<br>+74.8 |

TABLE II (Continued)

Evaluation of Test Compounds as Animal Growth Promoters

| Compound | Dosage (ppm) | Gain (grams) | % Gain Over Controls |
|---|---|---|---|
| 3-Bromo-5-[2-*tert*-butylamino)-1-hydroxyethyl]-anthranilic acid | 200<br>50 | 18.2<br>13.6 | +70.1<br>+27.1 |
| N-*tert*-butyl-3,5-dichloro-8-methoxy-4-methylaminophenethylamine hydrochloride | ·200<br>50 | 18.0<br>23.1 | +68.2<br>+115.9 |
| α-[(*tert*-butylamino)methyl]-3,5-dichloro-4-(hexylamino)benzyl alcohol | 200<br>50 | 19.6<br>20.7 | +83.2<br>+93.5 |
| 4-Amino-α-[(*tert*-butylamino)methyl]-3,5-dichlorobenzyl alcohol acetate | 200<br>50 | 14.4<br>18.7 | +34.6<br>+74.8 |
| β-(allyloxy)-4-amino-N-*tert*-butyl-3,5-dichlorophenethylamine | 200<br>50 | 21.5<br>19.6 | +100.9<br>+83.2 |
| 4-(allylamino)-α-[(*tert*-butylamino)methyl]-3,5-dichlorobenzyl alcohol | 200<br>50 | 20.2<br>21.9 | +88.8<br>+104.7 |
| 4′-[3-(*tert*-butylamino)-1-hydroxyethyl]-2′, 6′-dichloroacetanilide acetate (ester), hydrochloride | 200<br>50 | 25.8<br>16.2 | +141.1<br>+51.4 |
| α-[(*tert*-Butylamino)methyl]-3,5-dichloro-4-cyclohexylaminobenzyl alcohol | 100 | 23.0 | +115.0 |
| α-[(*tert*-Butylamino)methyl]-4-amino-3-chloro-5-methylbenzyl alcohol, hydrochloride | 200<br>50 | 16.5<br>19.7 | +54.2<br>+84.1 |

Example 2

Antilipogenic Evaluation of Test Compounds — Mouse Study

CFI female mice, 55 days old, are weighed in groups of 10 and allotted to cages to minimize weight variation among cages. Treatments are randomly assigned to cages.

Each of the treatments are tested in 3 replicates, i.e., in 3 cages of 10 mice each. There are 10 cages of 10 control mice each. Drugs are mixed in the diet at the dosage level indicated. Feed and water are offered ad libitum for 12-day test period. Feed spilled is collected during the test period. At the end of the test period, the collected feed is weighed and the mean feed consumption per cage of ten mice is determined for each treatment. The mice are weighed as a group of 10 and the weight gain determined. The mice are sacrificed by cervical dislocation. The right uterine fat pad of each mouse is removed. The fat pads for each cage of 10 mice are weighed as a unit.

Data obtained are reported in Table III. Data are reported as percent reduction in fat pad weight. Reduction in fat pad weights of animals is generally indicative of reduction of total body fat of the treated animals.

TABLE III

Antilipogenic Evaluation of Test Compounds — Mouse Study

| Compound | Dosage (ppm) | % Reduction in Fat Pad Weight vs Controls |
|---|---|---|
| α-[(*tert*-butylamino)methyl]-3,5-dichloro-4-dimethylamino benzyl alcohol | 200 | –46.1 |
| | 50 | –14.8 |
| Methyl-4-[2-(*tert*-butylamino)-1-hydroxyethyl]-2,6-dichlorocarbonilate | 50 | –23.5 |
| α-[(*tert*-butylamino)methyl]-3,5-dichloro-4-methylamino-benzyl alcohol | 200 | –51.0 |
| | 50 | –41.9 |
| 5-[2-*tert*-butylamino-1-hydroxyethyl]-3-chloroanthranilonitrile | 200 | –45.9 |
| | 50 | –10.4 |
| α-[(*tert*-butylamino)methyl]-1,3-dichloro-4-isopropylamino-benzyl alcohol | 200 | –52.5 |
| | 50 | –22.6 |
| | 12 | –6.3 |
| | 3 | –25.5 |
| Methyl-5-[2-(*tert*-butylamino)-1-hydroxyethyl]-3-chloro-anthranilate hydrochloride | 200 | –5.9 |
| | 50 | –5.8 |
| 5-[2-(*tert*-butylamino)-1-hydroxyethyl] anthranilonitrile | 200 | –41.5 |
| | 50 | –10.3 |
| 4-Amino-N-*tert*-butyl-3,5-dichloro-β-(methylthio) phenethylamine hydrochloride | 200 | –28.9 |
| | 50 | –16.2 |

Example 3

N-*tert*-butyl-3,5-dichloro-β-methoxy-4-methylaminophenethylamine hydrochloride

A 7 g sample of α-[(*tert*-butylamino)methyl]-3,5-dichloro-4-methylaminobenzyl alcohol is added to 70 ml of thionyl chloride under $N_2$ atmosphere and the mixture is stirred for two hours. Excess thionyl chloride is removed *in vacuo*, and the glassy residue is dissolved in 50 ml of methanol. The solution is stirred for 1.5 hours and evaporated to dryness. The residue is dissolved in 100 ml of $H_2O$ and extracted with 2 × 50 ml of $CH_2Cl_2$. The aqueous layer is neutralized with solid $NaHCO_3$9 and extracted with $CH_2Cl_2$. The extract is dried ($MgSO_4$) and evaporated to dryness *in vacuo* to give 4.1 g of semi-solid, which after trituration with ethyl ether affords 1.07 g of the title compound, mp 220—221°C. Similarly, the following ethers are prepared:

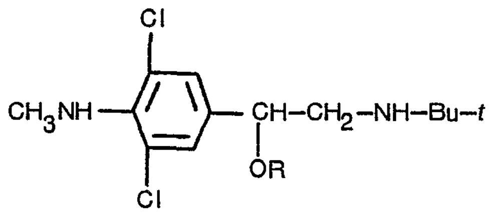

| Alcohol | R |
|---|---|
| ethanol | $C_2H_5$ |
| 1-propanol | 1-$C_3H_7$ |
| 2-propanol | 2-$C_3H_7$ |
| 1-butanol | 1-$C_4H_9$ |
| 2-butanol | 2-$C_4H_9$ |
| 1-hexanol | *n*-$C_6H_{13}$ |
| allyl alcohol | allyl |
| 4-methoxybenzyl alcohol | 4-methoxybenzyl |
| 4-chlorobenzyl alcohol | 4-chlorobenzyl |
| 4-nitrobenzyl alochol | 4-nitrobenzyl |
| 4-methylbenzyl alcohol | 4-methylbenzyl |
| 3,4-dimethylbenzyl alcohol | 3,4-dimethylbenzyl |
| 3,4-dimethoxybenzyl alcohol | 3,4-dimethoxybenzyl |
| 3,4-dichlorobenzyl alcohol | 3,4-dichlorobenzyl |
| 2-chlorobenzyl alcohol | 2-chlorobenzyl |
| 2-methylbenzyl alcohol | 2-methylbenzyl |

Example 4

In the manner described in Example 3, the following ethers are prepared by substituting the corresponding alcohols for methanol.

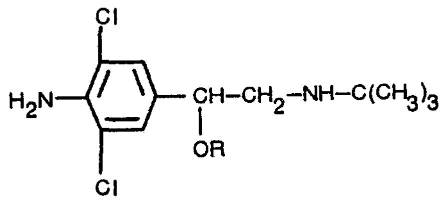

| R | mp°C |
|---|---|
| allyl | 57—59 |
| 4-methoxybenzyl | |
| 4-chlorobenzyl | |
| 4-nitrobenzyl | |
| 4-methylbenzyl | |
| 3,4-dimethylbenzyl | |
| 3,4-dimethoxybenzyl | |
| 3,4-dichlorophenyl | |
| 4-chlorophenyl | |
| 4-methoxyphenyl | |
| 4-methoxyphenyl | |
| 2-chlorophenyl | |
| 4-nitrophenyl | |

Example 5

N-*tert*-Butyl-3-chloro-5-cyano-β-methoxy-4-aminophenethylamine hydrochloride

In the manner described in Example 3, α-(*tert*-butylamino)methyl-4-amino-3-chloro-5-cyanobenzyl alcohol is converted into the title compound, and, similarly, the following are also prepared:

$$Ar—CH(OCH_3)—CH_2—NH—R—HCl$$

| Ar | R |
|---|---|
| 4-amino-3-chloro-5-trifluoromethylphenyl | *t*-butyl |
| 4-amino-3-chloro-5-trifluoromethylphenyl | *i*-propyl |
| 4-amino-3-chloro-5-$H_2N$—CO-phenyl | *t*-butyl |
| 4-amino-3-chloro-5-HO—CO-phenyl | *t*-butyl |
| 4-amino-3-chloro-5-methylphenyl | *t*-butyl |
| 4-amino-3-chloro-5-methoxyphenyl | *t*-butyl |
| 4-amino-3-chloro-5-nitrophenyl | *t*-butyl |
| 4-amino-3-chloro-5-$CH_3O$—CO-phenyl | *t*-butyl |
| 4-amino-5-cyano-phenyl | *t*-butyl |

Example 6

4-Amino-α-[(*tert*-butylamino)methyl]-3,5-dichlorobenzyl alcohol acetate

A mixture containing 1 g of 4-amino-α-[(*tert*-butylamino)methyl]-3,5-dichlorobenzyl alcohol in 35 ml of $CH_2Cl_2$ at 10—15°C is stirred, and 0.37 g of $Ac_2O$ and 0.5 ml of $Et_3N$ are added dropwise. The reaction mixture is then allowed to warm to room temperature, and the reaction is followed by thin-layer chromatography to completion. The mixture is evaporated to dryness *in vacuo*, and the yellow viscous liquid (1.5 g) is stirred with 50 ml of ethyl ether to afford a yellow solid (0.84 g), mp 128—131°C. This material is shown by nuclear magnetic resonance spectroscopy and by neutralization with alkali to be the acetic acid salt. On treating 100 mg of this salt in 30 ml of $CH_2Cl_2$ with 30 ml of 10% aqueous NaOH, the salt is neutralized. The $CH_2Cl_2$ solution is dried ($MgSO_4$) and evaporated to dryness *in vacuo* to afford the viscous title compound.

*Analysis:*

| | | | |
|---|---|---|---|
| Calcd for $C_{14}H_{20}O_2N_2Cl_2$: | C, 52.67; | H, 6.32; | N, 8.78; |
| Found: | C, 52.38; | H, 6.51; | N, 8.88. |

In the same manner, propionic anhydride, butyric anhydride, are allowed to react with 4-amino-α-[(*tert*-butylamino)methyl]-3,5-dichlorobenzyl alcohol (*A*) and α-[(*tert*-butylamino)methyl]-3,5-dichloro-4-methylaminobenzyl alcohol (*B*) respectively, to afford the propionate, butyrate and pivalate of *A* and *B*.

Example 7

The following esters are prepared by the method of Example 6 by using the appropriate acid anhydride.

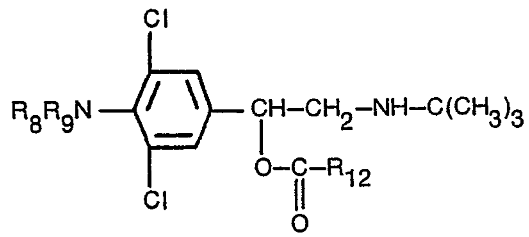

| $R_8$ | $R_9$ | $R_{12}$ |
|---|---|---|
| H | $CH_3$ | $CH_3$ |
| H | $C_2H_5$ | $CH_3$ |
| H | n-$C_3H_7$ | $CH_3$ |
| H | 2-$C_3H_7$ | $CH_3$ |
| H | allyl | $CH_3$ |
| $CH_3$ | $CH_3$ | $CH_3$ |
| H | $CH_3$ | $C_2H_5$ |
| H | $CH_3O$—CO— | $CH_3$ |
| H | $CH_3$ | n-$C_4H_9$ |
| $C_2H_5$ | $C_2H_5$ | $CH_3$ |
| n-$C_4H_9$ | n-$C_4H_9$ | $CH_3$ |

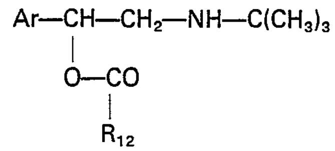

| Ar | $R_{12}$ |
|---|---|
| 3,5-dichlorophenyl | 2-$C_3H_7$ |
| 4-amino-3-chloro-5-cyanophenyl | $CH_3$ |
| 4-amino-3-chloro-5-trifluoromethylphenyl | $CH_3$ |
| 4-amino-3-chloro-5-$H_2NCO$-phenyl | $CH_3$ |
| 4-amino-3-chloro-5-HOOC-phenyl | $CH_3$ |
| 4-amino-3-chloro-5-methylphenyl | $CH_3$ |
| 4-amino-3-bromo-5-cyanophenyl | $CH_3$ |
| 4-amino-3-chloro-5-$CH_3OCO$-phenyl | $CH_3$ |
| 4-amino-5-cyanophenyl | *t*-$C_4H_9$ |

## Example 8

4-Acetamido-α-[(*tert*-butylamino)methyl]-3,5-dichlorobenzyl alcohol acetate

In 15 ml of $CH_2Cl_2$, 1.57 g of 4-acetamido-β-[(*tert*-butylamino)methyl]-3,5-dichlorobenzyl alcohol is suspended and stirred while 1.2 g of triethylamine in 30 ml of 30 ml of $CH_2Cl_2$ is added, followed by 0.7 g of acetic anhydride in 15 ml of $CH_2Cl_2$. The mixture is stirred for 20 hours and then is washed with 100 ml of 10% NaOH solution. The organic phase is separated, dried ($Na_2SO_4$) and evaporated to dryness *in vacuo*. The residue is dissolved in 30 ml of ethanol and a trace of $H_2O$ is added, followed by 10% HCl to acidify. The mixture is evaporated to dryness *in vacuo* and the residue is crystallized from acetone/hexane (30 ml/5 ml). This affords 1.35 g, mp. 254—257°C dec., of the title compound.

Similarly, by replacing acetic anhydride with propionic anhydride, butyric anhydride and pivalic anhydride, the corresponding propionate, butyrate and pivalate are prepared.

## Example 9

α-[(*tert*-butylamino)-methyl]3,5-dichloro-4-dimethylaminobenzyl alcohol

A mixture containing 50 g of *p*-fluoroacetophenone and 150 ml of 40% aqueous dimethylamine is warmed in a pressure bottle at 90—100°C. After two hours, a pale yellow oil is formed. The mixture is cooled, and the oil solidifies. The solid is collected and washed well with $H_2O$ to give 54.93 of *p*-dimethylaminoacetophenone, mp 101—103°C, after heptane recrystallization. A 72 g sample of this acetophenone is heated with 129 g of N-chlorosuccinimide in 700 ml of toluene to reflux temperature and maintained at this temperature for 35 minutes. The mixture is cooled and filtered. The filter cake is washed with 200 ml of toluene, and the filtrate and wash solution are evaporated to dryness *in vacuo* to afford 66 g of oil. This oil is chromatographed on $SiO_2$ with 40% hexane/$CH_2Cl_2$ to give 38.9 g of 3.5-dichloro-4-dimethylaminoacetophenone as a yellow oil. A 5.22 g sample of this oil is added portionwise to 2.75 g of $SeO_2$ in 20 ml of dioxane and 0.7 ml of $H_2O$ at 55—60°C. This mixture is heated at reflux temperature for 4.5 hours, cooled and filtered through siliceous earth. The filter cake is washed with 20 ml of dioxane. The dioxane solutions are cooled to 15°C and 2.77 g of *t*-butylamine is added dropwise to afford a tan precipitate. After stirring 15 minutes at room temperature, the mixture is diluted with 200 ml of ethanol, cooled to 50°C and 7 g of $NaBH_4$ is added portionwise. After 15 hours, the mixture is treated with 300—400 g of ice and 200 ml of $H_2O$ at below 10°C. The mixture is stirred to dissolve all solids and extracted with 300 ml of $CH_2Cl_2$. The $CH_2Cl_2$ layer is washed with 100 ml of $H_2O$, dried ($MgSO_4$) and evaporated to dryness *in vacuo* to give 5.6 g of orange oil. This oil is dissolved in ethyl ether, decolorized with activated carbon and concentrated to 15 ml. On cooling, crystals are obtained. The title product is collected as white crystals, mp 96—99°C.

The following compounds are prepared by the same method

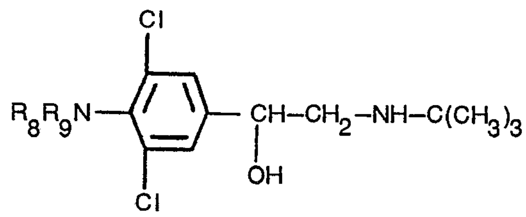

| $R_8$ | $R_9$ | mp °C |
|---|---|---|
| H | 1-$C_4H_9$ | oil |
| H | 1-$C_6H_{13}$ | 62—64 |
| H | $C_2H_5$ | 209 (HCl salt) |
| H | cyclopentyl | oil |
| H | cyclohexyl | 194—198 (HCl salt) |

Example 10

α-[(*tert*-butylamino)methyl]-3,5-dichloro-4-methylaminobenzyl alcohol

p-Methylaminoacetophenone is prepared and chlorinated by method described in Example 19 to give 3,5-dichloro-4-methylaminoacetophenone. This ketone (18 g) in 200 ml of $CHCl_3$ is stirred and 4.65 ml of $Br_2$ in 50 ml of $CHCl_3$ is added dropwise. After the addition is completed the mixture is stirred an additional 20 minutes and warmed to reflux temperature for 25 minutes. The mixture is cooled, 100 ml of $H_2O$ is added and saturated $Na_2CO_3$ solution is added carefully until the mixture is neutral. The $CHCl_3$ layer is separated and the aqueous layer is further extracted with 100 ml of $CH_2Cl_2$. The combined extracts are dried ($MgSO_4$) and evaporated to dryness to afford 16.3 g of the phenacyl bromide. This material (16 g) in 80 ml of EtOH is stirred at 12—15°C and 40 ml of *t*-butylamine is added dropwise. After the addition is completed the mixture is stirred for 10 minutes at 12—15°C and then cooled to 5° and 4 g of $NaBH_4$ is carefully added. After stirring for 0.5 hours, the mixture is allowed to warm to room temperature and stirring is continued for 0.75 hours. The mixture is poured on 300 ml of ice with stirring and the resulting mixture is extracted with 300 ml of $CH_2Cl_2$. The $CH_2Cl_2$ extract is dried ($MgSO_4$) and evaporated to dryness *in vacuo* to give a yellow oil. Trituration of this residue with ethyl ether affords 7.45 g of the title compound, which melts at 98—101°C after recrystallization from ethyl ether.

Example 11

5-[2-(*tert*-butylamino)-1-hydroxyethyl]anthranilonitrile

A mixture containing 48.86 g of *p*-aminoacetophenone in 490 ml of toluene is stirred while 64.5 g of N-bromosuccinimide is added in portions over 0.5 hours at below 40°C. After 15 minutes, the mixture is washed with $H_2O$ (4 × 100 ml). The solution is dried ($MgSO_4$) and evaporated to dryness to afford 70.53 g of 4-amino-3-bromoacetophenone, mp 59—62°C. A 35 g sample of this material in 180 ml of dry dimethylformamide is stirred and heated at reflux with 17.57 g of $Cu_2(CN)_2$ for 6 hours under $N_2$ atmosphere. Subsequently, 180 ml of $FeCl_2$/HCl solution (40 g $FeCl_3$.6 $H_2O$/10 ml concentrated HCl/60 ml $H_2$) is added and the mixture is heated for 20 minutes at 60—70°C and poured into 350 ml of $H_2O$. The aqueous mixture is extracted with $CH_2Cl_2$ and the extracts are washed with $H_2O$, saturated $NaHCO_3$ solution and $H_2O$, respectively. The $CH_2Cl_2$ solution is evaporated to dryness *in vacuo* and the residue is recrystallized from 95% EtOH to afford 14.25 g, mp 155—15°C, of 4-amino-3-cyanoacetophenone. A 4.8 g sample of this product in 100 ml of EtOAc and 100 ml of $CHCl_3$ containing 13.32 g of $CuBr_2$ is heated at reflux temperature for 20 minutes. The mixture is further heated after 20 ml of EtOH is added and then filtered while still hot. The filter cake is washed with 50 ml of hot 20% MeOH/$CH_2Cl_2$ and the combined organic solutions are evaporated to dryness *in vacuo*. The residue is stirred in 25 ml of $CH_2Cl_2$ and the solid is collected and washed with $CH_2Cl_2$ to give 8.08 g of the phenacyl bromide. This material is added to 50 ml of t-$BuNH_2$ in 100 ml of EtOH at 5° under $N_2$ atmosphere. After 10 minutes of stirring, the mixture is allowed to warm to 30°C to give a solution. This solution is cooled to 10° and 4 g of $NaBH_4$ is added in portions. After 45 minutes, the mixture is allowed to warm (42°C) and kept at 20°C until the exotherm subsides. The mixture is then evaporated to dryness and the residue is washed with $H_2O$. The residue is dried and treated with 200 ml of boiling MeOH and the hot MeOH solution is filtered. The filter cake is further washed with hot MeOH and the combined filtrates are concentrated to afford crystals. This solid is recrystallized from MeOH/2-PrOH to afford 2.08 g, mp 184—186°C, of the title compound.

In a similar manner, the following related compounds are prepared starting with the appropriate acetophenone:

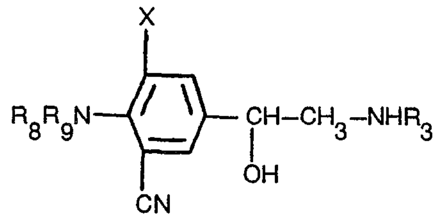

| $R_8$ | $R_9$ | $R_3$ | X |
|---|---|---|---|
| H | H | 2-$C_3H_7$ | H |
| H | $CH_3$ | *t*-butyl | H |
| $CH_3$ | $CH_3$ | *t*-butyl | H |
| H | $C_2H_5$ | *t*-butyl | H |
| H | n-$C_3H_7$ | *t*-butyl | H |
| J | 2-$C_3H_7$ | *t*-butyl | H |
| H | n-$C_4H_9$ | *t*-butyl | H |
| H | $CH_3$ | 2-$C_3H_7$ | H |
| $C_2H_5$ | $C_2H_5$ | *t*-butyl | Cl |
| *n*-$C_3H_7$ | *n*-$C_3H_7$ | *t*-butyl | Cl |
| *n*-$C_4H_9$ | *n*-$C_4H_9$ | *t*-butyl | Cl |

## Example 12

3-chloro-5-[2-(*tert*-butylamino)-1-hydroxyethyl]anthranilonitrile

In 100 ml of toluene, 5 g of 4-amino-3-cyanoacetophenone is heated at reflux temperature for 20 minutes with 4.2 g of N-chlorosuccinimide. The mixture is cooled and filtered. The filtrate is further heated at reflux temperature for 2 hours. The precipitate is collected and washed with $H_2O$. The remaining solid is treated with 0.75 ml of $Br_2$/14 ml of $CHCl_3$ added to 75 ml of $CHCl_3$ and 4.9 ml of EtOH. The mixture is evaporated to dryness and the residue is slurried with $CH_2Cl_2$, collected and washed with $CH_2Cl_2$ to afford 2.84 g of the phenacyl bromide. This material is allowed to react with *t*-$BuNH_2$ and reduced with $NaBH_4$ by the procedure of Example 11 to afford the title compound, mp 128—138°C.

In a similar manner, the following compounds are prepared:

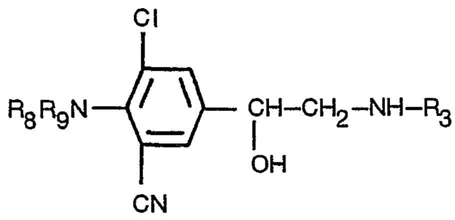

| $R_8$ | $R_9$ | $R_3$ |
|---|---|---|
| H | H | 2-propyl |
| H | $CH_3$ | *t*-butyl |
| $CH_3$ | $CH_3$ | *t*-butyl |
| H | $C_2H_5$ | *t*-butyl |
| H | 2-propyl | *t*-butyl |
| H | n-butyl | *t*-butyl |

Example 13

5-[2-(*tert*-butylamino)-1-hydroxyethyl]-3-chloro-anthranilic acid, methyl ester, hydrochloride

A mixture containing 1.36 g of 5-[2-(*tert*-butylamino)-1-hydroxyethyl]-3-chloroanthranilonitrile in 21 ml of 50% aqueous NaOH and 21 ml of EtOH is stirred under $N_2$ for 0.5 hours. The mixture is evaporated to remove EtOH and acidified to pH 3 and further evaporated to dryness *in vacuo*. The residue treated several times with MeOH and evaporated to dryness. The solid is then treated with a solution which is prepared from 40 ml of MeOH and 2 ml of acetyl chloride. After allowing to stand overnight, the mixture is filtered and the filtrate is evaporated to dryness. The filter cake is also washed with MeOH and added to preceding filtrate. The residue is dissolved in acetone, filtered, and evaporated to dryness. The solid is triturated with $Et_2O$ and filtered to give 1.49 g, mp 95—115°C, of the title compound.

In a similar manner, the following related esters are prepared:

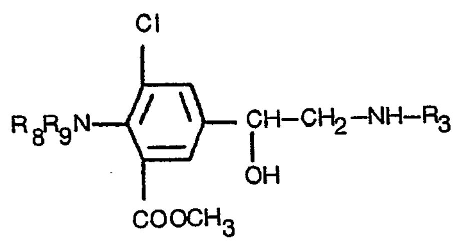

| $R_8$ | $R_9$ | $R_3$ |
|---|---|---|
| H | H | 2-propyl |
| H | $CH_3$ | *t*-butyl |
| $CH_3$ | $CH_3$ | *t*-butyl |
| H | $C_2H_5$ | *t*-butyl |
| H | *n*-propyl | *t*-butyl |
| H | n-butyl | *t*-butyl |
| H | allyl | *t*-butyl |
| $C_2H_5$ | $C_2H_5$ | *t*-butyl |
| *n*-$C_4H_9$ | *n*-$C_4H_9$ | *t*-butyl |
| *n*-$C_3H_7$ | *n*-$C_3H_7$ | *t*-butyl |

Example 14

2-Amino-3-bromo-5-[2-(*tert*-butylamino)-1-hydroxyethyl]benzamide

A mixture containing 1.02 g of 3-bromo-5-[2-(*tert*-butylamino)-1-hydroxyethyl)anthranilonitrile in 25 ml of $H_2O$, 5 ml of 50% NaOH and 30 ml of EtOH is stirred and heated at 55—65°C under $N_2$ atmosphere for 1.25 hours. The mixture is evaporated to remove EtOH and extracted with $CHCl_3$. The $CHCl_3$ extract is washed with 25 ml of 2% NaOH, dried ($MgSO_4$) and evaporated to dryness to afford 0.74 g. This solid is stirred with pentane and filtered to afford 0.6 g, mp 135—145°C, of the title compound.

Similarly, the following compounds are prepared:

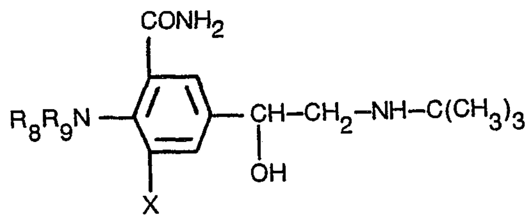

| $R_8$ | $R_9$ | X |
|---|---|---|
| H | $CH_3$ | Cl |
| H | $C_2H_5$ | Cl |
| $CH_3$ | $CH_3$ | Cl |
| H | 2-$C_3H_7$ | Cl |
| H | n-$C_4H_9$ | Cl |
| H | $CH_3$ | Br |
| $C_2H_5$ | $C_2H_5$ | Cl |
| *n*-$C_3H_7$ | *n*-$C_3H_7$ | Cl |
| *n*-$C_4H_9$ | *n*-$C_4H_9$ | Cl |

Example 15

4-amino-N-*tert*-butyl-3,5-dichloro-β-(methylthio)phenethylamine hydrochloride

In the manner described in Example 3, N-*tert*-butyl-3,5-dichloro-β-chloro-4-aminophenethylamine hydrochloride is prepared. An 11 g sample of this product is portionwise added to 5 ml of methyl mercaptan in 100 ml of dry ethylenedichloride at +10°C to 0°C. The mixture is stirred and allowed to rise gradually to room temperature over a four day period. The mixture is filtered, the filter cake is washed with ethylenedichloride (2 × 500 ml). The solid is dispersed in 200 ml of $H_2O$, cooled to 5°C and basified with 6N NaOH solution to give a white oil, which is extracted with $CH_2Cl_2$ (3 × 100 ml). The $CH_2Cl_2$ (3 × 100 ml). The $CH_2Cl_2$ extract is dried ($MgSO_4$) and evaporated to dryness to give 6.41 g of dark green oil. This oil is stirred in HCl/isopropanol and the mixture is evaporated to dryness. The residue is stirred in 35 ml of ethyl ether for 16 hours and filtered to give 3.63 g, mp 178—181°C dec. This solid is heated in refluxing ethyl acetate and filtered to give 2.07 g, mp 188—193°C. Recrystallization from 75 ml of ethylenedichloride affords 1.45 g of the title compound, mp 191—196°C.

The title compound is also obtained by adding 5—10 fold excess of sodium mercaptide in tetrahydrofuran at 0—10°C and by following the above workup.

Example 16

In the same manner as described in Example 15, the following thioesters are prepared by substituting methyl mercaptan with the corresponding mercaptans:

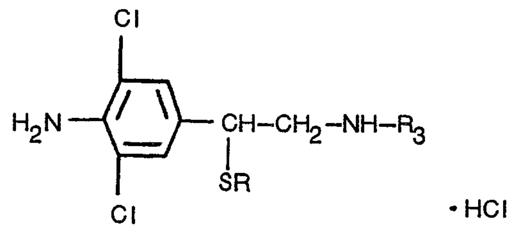

| R | $R_3$ |
|---|---|
| methyl | 2-propyl |
| ethyl | *t*-butyl |
| 2-propyl | *t*-butyl |
| *n*-butyl | *t*-butyl |
| *t*-butyl | *t*-butyl |
| *n*-hexyl | *t*-butyl |
| phenyl | *t*-butyl |
| benzyl | 2-propyl |

Example 17

In the manner described in Example 15, substitution of corresponding chloro compound for N-*tert*-butyl-3,5-dichloro-β-chloro-4-aminophenethylamine hydrochloride and adding the appropriate mercaptens afford the following thioethers:

$$Ar—\underset{\displaystyle Cl}{\underset{|}{CH}}—CH_2—NH—R_3 + RSH \rightarrow Ar—\underset{\displaystyle SR}{\underset{|}{CH}}—CH_2—NHR_3$$

| Ar | R | $R_3$ |
|---|---|---|
| 4-amino-3-cyanophenyl | methyl | 2-propyl |
| 4-methylamino-3,5-dichlorophenyl | methyl | *t*-butyl |
| 4-amino-3-chloro-5-trifluoromethyl | methyl | *t*-butyl |
| 4-amino-3-chloro-5-cyanophenyl | methyl | *t*-butyl |
| 4-amino-3-chloro-5-cyanophenyl | ethyl | *t*-butyl |
| 4-acetamido-3,5-dichlorophenyl | methyl | *t*-butyl |
| 4-amino-3-chloro-5-$H_2NCO$-phenyl | methyl | *t*-butyl |
| 4-amino-3-chloro-5-HOCO-phenyl | methyl | *t*-butyl |
| 4-amino-3-chloro-5-methylphenyl | ethyl | *t*-butyl |
| 4-amino-3-chloro-5-methoxyphenyl | n-butyl | *t*-butyl |
| 4-amino-3-chloro-5-nitrophenyl | methyl | *t*-butyl |
| 4-amino-3-chloro-5-$CH_3O$—CO-phenyl | methyl | *t*-butyl |

Example 18

α-[(*tert*-butylamino)methyl]-3,5-dichloro-4-diethylaminobenzyl alcohol

In the manner described in Example 9, 3,5-dichloro-4-diethylaminoacetophenone is oxidized with $SeO_2$ and reductively alkylated with *t*-$BuNH_2/NaBH_4$ to afford the title compound, mp 93—96°C.

Similarly, α-[(*tert*-butylamino)methyl]-3,5-dichloro-4-(*n*-dipropyl)aminobenzyl alcohol and α-[(*tert*-butylamino)methyl]-3,5-dichloro-4-(*n*-dibutyl)aminobenzyl alcohol are prepared.

Example 19

4-(Allyamino)-α-[(*tert*-butylamino)methyl]-3,5-dichlorobenzyl alcohol

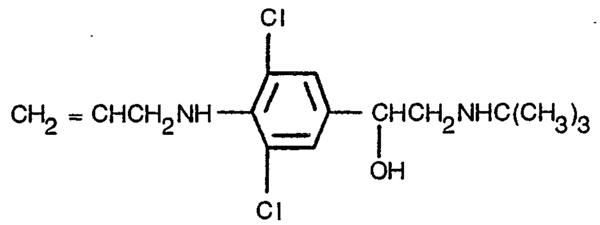

(a) Triethylamine (17.0 g, 0.168 g, 0.168 mol) is added in one portion to allyl bromide (105.9 g, 0.875 mol) under a nitrogen atmosphere. The resulting white emulsion gives an exotherm to 70°C and becomes a thick white solid mass within 5 minutes. The solution formed with the addition of ~100 ml of DMF is stirred for 1 hour at 70—95°C. A solution of 4'-amino-2-bromo-3',5'-dichloroacetophenone (25.0 g, 0.088 mol) in 50 ml of DMF is added in one portion and the resulting brown reaction mixture is maintained at 80—90°C for 2 hours. The progress of the reaction is frequently checked by thin layer chromatography ($SiO_2/CH_2Cl_2/$ hexanes (1/1)) since prolonged heating results in the decomposition of both starting material and products. The reaction mixture is poured into 1.51 of $H_2O$ and is stirred for 0.5 hours. After a second aqueous trituration, the residual brown semi-solids are stirred with ~150 ml of CCl for 0.5 hours to form a suspension. The yellowish-brown solids are collected by filtration and are air dried to give 14.9 g (59.6%) of recovered phenacyl bromide starting material. The $CCl_4$ filtrate is stirred with $MgSO_4$, filtered and concentrated to yield 9.42 g of a brown syrup. Gradient elution (hexanes/$CH_2/Cl_2$) (10/0 → 8/2 flash chromatography on a 9" × 2" column of Silica Gel 60 gives two major fractions:

(A) 1.82 g (5.7%) of a faster moving amber syrup, identified as 2-bromo-3',5'-dichloro-4'-diallylamino-acetophenone by IR (neat) 1680 $cm^{-1}$; NMR ($CDCl_3$) & 7.93 (s, 2, AR—H), 6.25—5.55 (complex m, 2, CH=), 5.40—4.95 (complex m, 4, $CH_2$), 4.40 (s, 2, $CH_2Br$) and 3.87 (m resembling d, 4, J=6Hz, $CH_2N$); chemical ionization mass spectrum $(M + H)^+$ = 3.62; and

(B) 3.49 g (12.2%) of a slower moving brown syrup, identified as 4'-(allyamino)-2-bromo-3',5'-dichloro-acetophenone by IR (neat) 3330, 1670 $cm^{-1}$; NMR ($CDCl_3$) & 7.83 (s, 2, AR—H), 6.35—5.65 (complex m, 1, CH=), 5.50—5.00 (complex m, 2, $CH_2$=), 4,84 (br, t, 1, NH), 4.37 (s, 2, $CH_2Br$) and 4.20 (br, m, 2, $CH_2N$); chemical ionization mass spectrum $(M + H)^+$ = 322.

(b) A solution of 4'-(allylamino)-2-bromo-3',5'-dichloroacetophenone (2.88 g, 8.92 mmol) in 10 ml is added dropwise over 1 hour to a stirred solution of *t*-butylamine (1.34 g, 18.3 mmol) in 29 ml of THF. The reaction temperature is maintained at −24°—13° by cooling a dry ice-$CCl_4$ bath. The resulting amber suspension is warmed to room temperature over 30 minutes and is stirred at 21—22°C for 1.5 hours. Sodium Cyanoborohydride (2.80 g, 44.6 mmol) is added in two portions over 5 minutes to give a thick tan suspension with an exotherm from 22—25°C. Glacial acetic acid (~10 ml) is added dropwise to gradually form a yellow solution which is stirred at room temperature for 3 days. The reaction mixture is poured into a solution of 100 ml of $H_2O$ and 100 ml of saturated aqueous NaCl which is then adjusted to pH7 with 10% $Na_2CO_3$ and extracted three times with $Et_2O$. The combined extracts are shaken with two portions of diluted aqueous HCl which are combined, neutralized with 10% $Na_2CO_3$ to pH8 and extracted three times with $Et_2O$. After stirring the combined extracts with anh. $K_2CO_3$, the pale yellow-green solution is filtered and concentrated to yield 2.04 g (72.1%) of pale yellow syrup, identified as 4-(allylamino)-α-[(*tert*-butylamino)-methyl]-3,5-dichlorobenzyl alcohol by IR (neat) 3400 $cm^{-1}$; NMR ($CDCl_3$) & 7.32 (s, 2, Ar—H), 6.35—5.60 (complex m, 1, CH=), 5.45—4.95 (complex m, 2, $CH_2$=), 4.52 (d of d, 1, Ar—CH), 3.97 (overlapping m, 3, Ar—$NHCH_2$), 3.03 (br s, 2, NH and OH), 2.68 (m, 2, $CH_2N$) and 1.13 (s, 9, $C(CH_3)_3$); chemical ionization mass spectrum $(M + M)^+$ = 3.17. The $CH_2Cl_2/CH_3OH$/conc. $NH_4OH$ (80/19/1)) shows one major spot ($R_f$ = 0.6) with nine trace impurities. The syrup gradually crystallizes to a tan solid on standing.

Example 20

α-[(*tert*-butylamino)methyl]-3,5-dichloro-4-diallylaminobenzyl alcohol

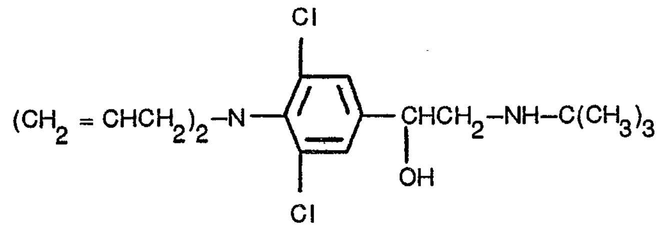

The title compound is prepared using a starting material fraction (A) of Example 19 (a) and the procedure described in Example 19 (b). The pale yellow syrup, which gradually crystallizes on standing, is identified by IR (neat) 3300 and 1630 $cm^{-1}$; NMR ($CDCl_3$) & 7.26 (s, 2, AR—H), 6.23—5.54 (complex m, 2, CH=), 5.32—4.87 (complex m, 4, $CH_2$=), 4.48 (m, 1, Ar—CH), 3.78 (m resembling d, 4, J—6Hz, Ar—$NCH_2$), 3.4—2.0 (br, s, 2, NH and OH), 2.62 (m, 2, $CH_2N$) and 1.13 (s, 9, $C(CH_3)_3$); chemical ionization mass spectrum $(M + H)^+ = 357$, corresponding to that expected of the title compound.

**Claims**

1. A method for enhancing the growth rate and for reducing the fat deposition in meat-producing animals by administering to said animals a compound of the following formula, at selected levels of administration,

(I) 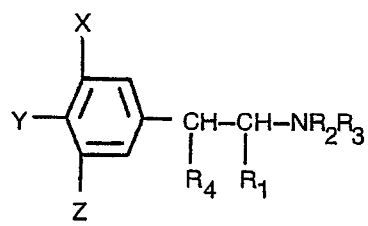

wherein

X is hydrogen or halogen;

Y is hydrogen, $NR_8R_9$ or $NHCOR_5$;

Z is halogen, CN, $CF_3$, $COOR_1$, $CONH_2$, methyl, methoxy or $NO_2$;

$R_1$ is hydrogen, $C_{1-4}$alkyl;

$R_2$ is hydrogen, $C_{1-6}$alkyl or $C_3$—$C_4$-alkenyl;

$R_3$ is hydrogen $C_1$—$C_6$alkyl, $C_3$—$C_6$cycloalkyl, $C_3$—$C_4$-alkenyl, phenyl, 2-hydroxyethyl, α,α-dimethylphenethyl or benzyl;

$R_4$ is OH, $OR_6$ or $SR_{11}$;

$R_5$ is hydrogen, $C_1$—$C_4$-alkyl, $C_1$—$C_4$-alkoxy or

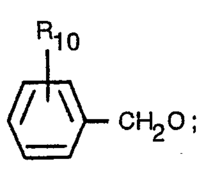

$R_6$ is $C_1$—$C_6$alkyl, $C_2$—$C_5$ alkanoyl,

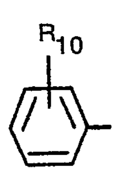 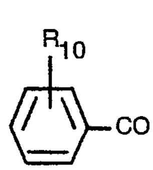 or 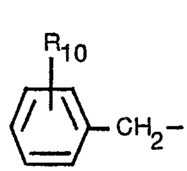 or $C_3$—$C_4$-alkenyl;

$R_8$ is hydrogen, $C_1$—$C_4$-alkyl or $C_3$—$C_4$-alkenyl;
$R_9$ is hydrogen, $C_1$—$C_6$ alkyl, $C_4$—$C_6$ cycloalkyl or $C_3$—$C_4$ alkenyl;
$R_{10}$ is chloro, dichloro, methyl, dimethyl, methoxy, dimethoxy or nitro;
$R_{11}$ is $C_1$—$C_6$-alkyl, phenyl, or benzyl;
with the privosos that
when $R_3$ is phenyl, 2-hydroxyethyl, α,α-dimethylphenethyl, $C_3$—$C_6$ cycloalkyl or benzyl $R_2$ is hydrogen; and
when $R_3$ is 2-hydroxyethyl, $R_4$ is hydroxyl; and
when $R_6$ is alkanoyl or

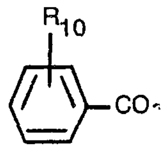

$R_2$ and $R_3$ are substituents other than hydrogen, except when $R_3$ is an alkyl or a substituted alkyl group which contains a tertiary carbon attached to the nitrogen; and
when Y is hydrogen, X and Z are halogen, and $R_2$ is hydrogen and $R_3$ is isopropyl, 2-butyl or *t*-butyl; and
when $R_8$ is $C_1$—$C_4$ alkyl or $C_3$—$C_4$ alkenyl, $R_9$ is hydrogen, $C_1$—$C_4$ alkyl or $C_3$—$C_4$ alkenyl; and
that at least one of X and Y represents a substituent other than hydrogen; and
when Z is a group other than halogen, Y is $NR_8R_9$ or $NHCOR_5$; and
further provided that when Y is hydrogen, $NH_2$ or $NHCOR_5$, and Z is halogen then $R_4$ cannot be OH or $OR_6$, wherein $R_6$ is $C_1$—$C_6$ alkyl;
racemic mixtures of the above identified compounds and the optically active isomers, and non-toxic, pharmacologically acceptable acid addition salts thereof.

2. A method according to Claim 1, characterized in that 5-[1-hydroxy-2-(isopropylamino)ethyl] anthranilonitrile is administered.

3. A method according to Claim 1, characterized in that 5-[2-(*tert*-butylamino)-1-hydroxyethyl]-N-ethyl-anthranilotrile is administered.

4. A method according to Claim 1, characterized in that 5-[2-(*tert*-butylamino)-1-hydroxyethyl] anthranilonitrile is administered.

**Patentansprüche**

1. Verfahren zur Steigerung der Wachstumsrate und zur Reduzierung der Fettablagerung bei fleischerzeugenden Tieren durch Verabreichung einer Verbindung der folgenden Formel mit ausgewählten Verabreichungspegeln an die Tiere

(I) 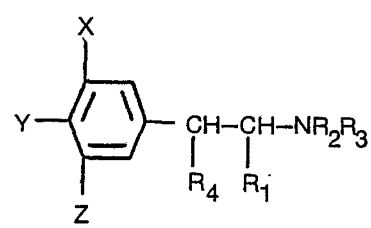

wobei
X für Wasserstoff oder Halogen steht;
Y für Wasserswtoff, $NR_8R_9$ oder $NHCOR_5$ steht;
Z für Halogen, CN, $CF_2$, $COOR_1$, $CONH_2$, Methyl, Methoxy oder $NO_2$ steht;
$R_1$ für Wasserstoff, $C_1$—$C_4$-Alkyl steht;
$R_2$ für Wasserstoff, $C_1$—$C_6$-alkyl oder $C_3$—$C_4$-Alkenyl steht;
$R_3$ für Wasserstoff, $C_1$—$C_6$-Alkyl, $C_3$—$C_6$-Cycloalkyl, $C_3$—$C_4$-Alkenyl, Phenyl, 2-Hydroxyäthyl, α,α-Dimethylphenäthyl oder Benzyl steht;
$R_4$ für OH, $OR_6$ oder $SR_{11}$ steht;

$R_5$ für Wasserstoff, $C_{1-4}$-Alkyl, $C_1$—$C_4$-Alkoxy oder

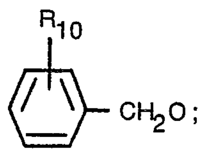

steht;

$R_6$ für $C_1$—$C_6$-Alkyl, $C_2$—$C_5$-Alkanoyl,

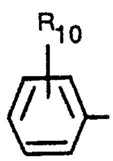 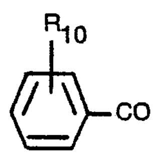 oder 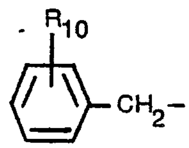

oder $C_3$—$C_4$-alkenyl;

$R_8$ für Wasserstoff, $C_1$—$C_4$-Alkyl oder $C_3$—$C_4$-Alkenyl steht;

$R_9$ für Wasserstoff, $C_1$—$C_6$-Alkyl, $C_4$—$C_6$-Cycloalkyl oder $C_3$—$C_4$-Alkenyl steht;

$R_{10}$ für Chlor, Dichlor, Methyl, Dimethyl, Methoxy, Dimethoxy oder Nitro steht;

$R_{11}$ für $C_1$—$C_6$-Alkyl, Phenyl oder Benzyl steht;

mit den Maßgaben, daß

falls $R_3$ für Phenyl, 2-Hydroxyäthyl, α,α-Dimethylphenäthyl, $C_3$—$C_6$-Cycloalkyl oder Benzyl steht, $R_2$ für Wasserstoff steht; und

falls $R_3$ für 2-Hydroxyäthyl steht, $R_4$ für Hydroxyl steht, und

falls $R_6$ für Alkanoyl oder

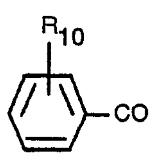

steht, $R_2$ und $R_3$ für andere Substituenten als Wasserstoff stehen, ausgenommen den Fall, daß $R_3$ eine Alkyl oder eine substituierte Alkylgruppe bedeutet, welche ein tertiäres Kohlenstoffatom, gebunden an den Stickstoff, enthält; und

falls Y für Wasserstoff steht, X und Z für Halogen stehen und $R_2$ für Wasserstoff steht und $R_3$ für Isopropyl, 2-Butyl oder *t*-Butyl steht; und

falls $R_8$ für $C_1$—$C_4$-Alkyl oder $C_3$—$C_4$-Alkenyl steht, $R_9$ für Wasserstoff, $C_1$—$C_4$-Alkyl oder $C_3$—$C_4$-Alkenyl steht; und

daß mindestens einer von X und Y für einen anderen Substituenten als Wasserstoff steht; und

falls Z für eine andere Gruppe als Halogen steht, Y für $NR_8R_9$ oder $NHCOR_5$ steht; und

mit der weiteren Maßgabe, daß falls Y für Wasserstoff, $NH_2$ oder $NHCOR_5$ steht, und Z für Halogen steht, $R_4$ nicht OH oder $OR_6$ sein kann, wobei $R_6$ für $C_1$—$C_6$-Alkyl steht;

racemische Mischungen der oben identifizierten Verbindungen und die optisch aktiven Isomeren und nichttoxische, pharmakologische akzeptable Säureadditionssalz derselben.

2. Verfahren gemäß Anspruch 1, dadurch gekennzeichnet, daß 5-[1-Hydroxy-2-(isopropylamino)äthyl]-anthranilonitril verabreicht.

3. Verfahren gemäß Anspruch 1, dadurch gekennzeichnet, daß 5-[2-(*tert*-Butylamino)-1-hydroxyäthyl]-N-äthylanthranilonitril verabreicht.

4. Verfahren gemäß Anspruch 1, dadurch gekennzeichnet, daß 5-[2-(*tert*-Butylamino)-1-hydroxyethyl]-anthranilonitril verabreicht.

**Revendications**

1. Procédépour accroître le taux de croissance et réduire le dépôt de graisses dans les animaux produisant de la viande par administration aux animaux, à des niveaux d'administration choisis, d'un compose' de formule suivante:

(I) 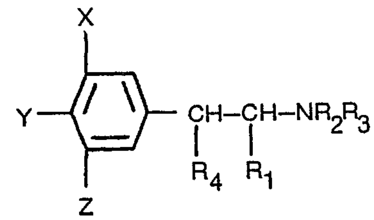

dans laquelle

X est un hydrogène ou halogène;

Y est un hydrogène, $NR_8R_9$ ou $NHCOR_5$;

Z est un halogène, CN, $CF_3$, $COOR_1$, $CONH_2$, méthyle, méthoxy ou $NO_2$;

$R_1$ est un hydrogène, $(C_{1-4})$alkyle;

$R_2$ est un hydrogène, $(C_{1-6})$ alkyle ou $(C_3—C_4)$-alkényle;

$R_3$ est un hydrogène, $(C_1—C_6)$-alkyle, $(C_3—C_6)$-cycloalkyle, $(C_3—C_4)$-alkényle, phényle, 2-hydroxyéthyle, α,α-diméthylphénéthyle ou benzyle;

$R_4$ est OH, $OR_6$ ou $SR_{11}$;

$R_5$ est un hydrogène, $(C_{1-4})$-alkyle, $(C_1—C_4)$alkoxy ou

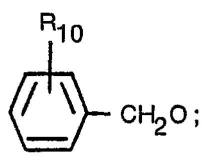

$R_6$ est un $C_1—C_6$ alkyle, $(C_2—C_5)$alcanoyle,

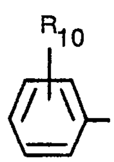 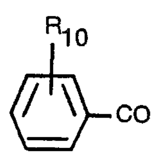 ou 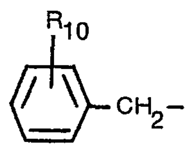

ou $C_3—C_4$-alcényle;

$R_8$ est un hydrogène, $(C_1—C_4)$-alkyle ou $(C_3—C_4)$alcényle;

$R_9$ est un hydrogène, $(C_1—C_6)$alkyle, $(C_4—C_6)$ cycloalkyle ou $(C_3—C_4)$ alcényle;

$R_{10}$ est un chloro, dichloro, méthyle, diméthyle, méthoxy, diméthoxy ou nitro;

$R_{11}$ est $(C_1—C_6)$-alkyle, phényle ou benzyle;

sous réserve que

lorsque $R_3$ est un phényle, 2-hydroxyéthyle, αα-diméthylphénétyle, $(C_3—C_6)$ cycloalkyle ou benzyle, $R_2$ est un hydrogène; et

lorsque $R_3$ est 2-hydroxyéthyle, $R_4$ est hydroxyle, et

lorsque $R_6$ est alcanoyle ou

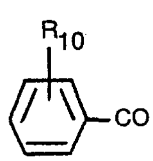

$R_2$ et $R_3$ sont des substituants autres que l'hydrogène, excepté lorsque $R_3$ est un groupe alkyle ou un groupe alkyle substitué qui contient un carbone tertiaire attaché à l'azote; et

lorsque Y est un hydrogène, X et Z sont des halogènes et $R_2$ est un hydrogène et $R_3$ est un isopropyle, 2 butyle ou *t*-butyle; et

lorsque $R_8$ est un ($C_1$—$C_4$)alkyle ou ($C_3$—$C_4$) à alcényle, $R_9$ est un hydrogène, ($C_1$—$C_4$)alkyle ou ($C_3$—$C_4$) alcényle; et

qu'au moins l'un de X et Y représente un substituant autre qu'un hydrogène, et

lorsque Z est un groupe autre qu'un halogène, Y est $NR_8R_9$ ou $NHCOR_5$; et

de plus sous réserve que lorsque Y est un hydrogène, $NH_2$ ou $NHCOR_5$, et Z est un halogène alors $R_4$ ne peut être OH oui $OR_6$, $R_6$ étant un ($C_1$—$C_6$)alkyle;

les mélanges racémiques de ces composés et leurs isomères optiquement actifs, et leurs sels d'addition à des acides pharmacologiquement acceptables.

2. Procédé selon la revendication 1, caractérisé en ce que l'on administre le 5-[1-hydroxy-2-(isopropilamino)ethyl] anthranilo-nitrile.

3. Procédé selon la revendication 1, caractérisé en ce que l'on administre le 5-[2-(*tert*-butylamino)-1-hydroxyéthyl]-N-éthylanthranilonitrile.

4. Procédé selon la revendication 1, caractérisé en ce que l'on administre le 5-[2-(*tert*-butylamino)-1-hydroxyéthyl] anthranilonitrile.